# EUROPÄISCHE PATENTANMELDUNG

(11) **EP 4 813 966 A1**
(43) Veröffentlichungstag der Anmeldung: **30.09.2026**
(21) Anmeldenummer: 25166810.9
(22) Anmeldetag: 27.03.2025
(51) Int. Cl.: C07C 43/11, C07C 41/03, C07C 41/38

(54) **ABTRENNUNG VON POLYETHYLENGLYKOLEN AUS ETHOXYLATEN**

(71) Anmelder: BASF SE, 67056 Ludwigshafen am Rhein (DE)
(72) Erfinder: HANSCH, Markus, 67056 Ludwigshafen am Rhein (DE); BERG, Silvia, 67056 Ludwigshafen am Rhein (DE); CARVALHO, Susan, 67056 Ludwigshafen am Rhein (DE); DECKERT, Petra, 67056 Ludwigshafen am Rhein (DE); BITTNER, Christian, 67056 Ludwigshafen am Rhein (DE)
(74) Vertreter: BASF IP Association

(57) **Zusammenfassung**

Die vorliegende Erfindung betrifft ein Verfahren zur Abtrennung von Oligo- und Polyethylenglykolen aus ethoxylierten Alkanolen.

## Beschreibung

Die vorliegende Erfindung betrifft ein Verfahren zur Abtrennung von Oligo- und Polyethylenglykolen aus ethoxylierten Alkanolen.

Die Ethoxylierung von Alkanolen erfolgt industriell meist durch Umsetzung der entsprechenden Alkanole mit Ethylenoxid unter Katalyse von Basen, wie beispielsweise Natrium- oder Kaliumhydroxid. Durch diese eingesetzten Basen oder als begleitender Inhaltsstoff im Alkanol wird Wasser in die Reaktion eingeführt, das zur Bildung von Oligo- und Polyethylenglykolen führt. Diese Oligo- und Polyethylenglykole weisen eine höhere Hydrophilie auf als die eigentlichen Zielprodukte.

In vielen Anwendungen ist die Anwesenheit der Oligo- und Polyethylenglykole nicht nachteilig, da die ethoxylierten Alkanole z.B. für Anwendungen in Tensiden oder Reinigungs- und Waschmitteln in der Anwendung ohnehin mit Polyethylenglykolen versetzt werden.

Einerseits ist jedoch das in den Oligoethylenglykolen enthaltene Diethylenglykol Ausgangsstoff für die Bildung des toxikologisch bedenklichen Dioxan, andererseits führen die hydrophilen höheren Ethylenglykole in den ethoxylierten Alkanolen zu einer Trübung. Ferner werden gerade bei niedrig-ethoxylierten Alkanolen, bei denen der hydrophobe Charakter des Alkanols ausgeprägt ist, die Eigenschaften des ethoxylierten Alkanols durch die Anwesenheit der hydrophilen Polyethylenglykole stark beeinträchtigt, da dadurch die Hydrophilie-Hydrophobie-Balance des Produktes beeinflusst wird.

Aufgabe der vorliegenden Erfindung war es, ein Verfahren zur Entfernung von Oligo- und Polyethylenglykolen aus ein- bis dreifach ethoxylierten C₈-C₁₄-Alkanolen zur Verfügung zu stellen, besonders solcher, die aus der basenkatalysierten Ethoxylierung erhalten worden sind.

DE 828839 beschreibt die Abtrennung von Polyglykolen aus Reaktionsgemischen der Alkoxylierung von Alkoholen, Säuren, Phenolen, Alkylphenolen und Naphtholen mit 20 bis 400% Wasser bei erhöhter Temperatur. Die explizit offenbarten Beispiele verwenden 50 bis 200 Gew% Wasser bezogen auf das Alkoxylat, wobei die Alkoxylate strukturell stark unterschiedlich sind, ethoxylierte Alkohole jedoch nicht explizit offenbart werden.

WO 2021/262439 A2 beschreibt die Extraktion von Polyethylenglykolen aus Fettalkoholethoxylaten, wobei mindestens 50 Gew% Wasser zur Extraktion eingesetzt werden.

Derartig hohe Mengen an Wasser haben den Nachteil, dass große Mengen an hydrophilem Wunschprodukt ebenfalls in die Wasserphase übergehen und somit verloren gehen.

Anstelle der Extraktion mit Wasser wird in WO 2021/262439 A2 auch die Extraktion mit elektrolythaltigen Lösungen, beispielsweise wäßriger Kochsalz-Lösung vorgeschlagen, was die Phasentrennung erleichtert.

Dies hat jedoch den Nachteil, dass Spuren des Elektrolyts in die organische Phase des Fettalkoholethoxylates übergehen, was beispielsweise bei Chloriden in späteren Anwendungen zu Korrosion führen kann.

EP 43963 A1 beschreibt die Ethoxylierung von primären Monoalkoholen mit Friedel-Crafts- oder sauren Katalysatoren und anschließender basischer Wäsche. Die Wäsche dient zur Entfernung des sauren Katalysators, Polyethylenglykole verbleiben ausdrücklich im Reaktionsgemisch.

EP 1015404 B1 beschreibt die Herstellung Randompolymerisaten von Fettalkoholen mit Ethylenoxid und Propylenoxid. Es wird darauf hingewiesen, dass als Nebenprodukte Polyethylenglykole gebildet werden. Diese ließen "sich zwar prinzipiell durch Extraktion mit geeigneten Lösemitteln wie Wasser entfernen, aber hierzu bedarf es einen weiteren Prozeßschritt, der sehr zeitaufwendig und zudem nicht universell anwendbar ist."

Eine Extraktion ist also mit Nachteilen verbunden und nicht notwendigerweise von einem Produkt auf das andere übertragbar.

Die Aufgabe wird gelöst durch ein Verfahren zur Abtrennung von Oligo- und Polyethylenglykolen der Formel

HO-[-CH₂-CH₂-O]ₙ-H

worin n eine rationale Zahl von mindestens 2, bevorzugt von 2 bis 30 und besonders bevorzugt von 2 bis 20 ist
aus ethoxylierten Alkanolen der Formel

   R¹-O-[-CH₂-CH₂-O-]ₘ-H

   worin
   R¹ geradkettiges oder verzweigtes, bevorzugt geradkettiges C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
   m eine rationale Zahl von 1 bis 3
   ist, in dem man
      (i) das Gemisch aus Oligo- und Polyethylenglykolen und ethoxylierten Alkanolen bei einer Temperatur von Umgebungstemperatur bis 90 °C mit dem 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase vermischt,
      (ii) die Phasen bei einer Temperatur von 30 bis 90 °C entmischen lässt,
      (iii) die organische von der wäßrigen Phase trennt und
      (iv) optional den Wassergehalt der organischen Phase verringert,
   dadurch gekennzeichnet, dass man die Extraktion in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

Eine bevorzugte Ausführungsform der vorliegenden Erfindung besteht darin, den Gehalt an gelöstem Sauerstoff im ethoxylierten Alkanol und/oder im Wasser vor Einsatz in die Extraktion zu verringern, besonders vor Erwärmen auf die Temperatur, bei der die Extraktion durchgeführt wird.

Der Erfindung liegt die Beobachtung zugrunde, dass sich aus dem ethoxylierten Alkanol bei thermischer Belastung in Gegenwart von Sauerstoff Oligo- und Polyethylenglykole bilden.

Die vorliegende Erfindung hat den Vorteil, dass sie die Verluste an dem ethoxyliertem Alkanol als Wertprodukt niedrig hält und gleichzeitig sowohl Oligo- und Polyethylenglykole als auch (Erd)Alkalimetallionen aus dem für die Ethoxylierung verwendeten Katalysator entfernt werden können, wenn die Ethoxylierung des Alkanols in Gegenwart mindestens eines basischen Salzes erfolgt war.

Durch den Einsatz von Wasser anstelle von z.B. Kochsalzlösung wird der Eintrag von Chlorid in das Wertprodukt vermieden, so dass das so erhaltene Produkt auch für solche Anwendungen geeignet ist, in denen Korrosion oder Verbrennungsvorgänge eine Rolle spielen.

Demzufolge ist ein weiterer Gegenstand der vorliegenden Erfindung ein Verfahren zur Herstellung von ethoxylierten Alkanolen der Formel

R¹-O-[-CH₂-CH₂-O-]ₘ-H

worin
R¹ geradkettiges oder verzweigtes C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
m eine rationale Zahl von 1 bis 3
mit einem verringerten Gehalt an Oligo- und Polyethylenglykolen der Formel

   HO-[-CH₂-CH₂-O]ₙ-H
worin n eine rationale Zahl von mindestens 2, bevorzugt von 2 bis 30 und besonders bevorzugt von 2 bis 20 ist,
und einem gleichzeitig verringerten Gehalt an (Erd)Alkalimetallionen,
dadurch gekennzeichnet, daß man
   (I) ein Alkanol R¹-OH mit mindestens m Äquivalenten Ethylenoxid in Gegenwart mindestens eines basischen Salzes mindestens eines (Erd)Alkalimetalls sowie in Gegenwart von Wasser bei einer Temperatur von 20 bis 200 °C unter Erhalt eines Gemisches aus Oligo- und Polyethylenglykolen und ethoxylierten Alkanolen miteinander reagieren lässt und
   (II) das so erhaltene Gemisch einer Aufreinigung unterwirft, in der man
   (i) das Gemisch aus Oligo- und Polyethylenglykolen und ethoxylierten Alkanolen bei einer Temperatur von Umgebungstemperatur bis 90 °C mit dem 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase vermischt,
   (ii) die Phasen bei einer Temperatur von 30 bis 90 °C entmischen lässt,
   (iii) die organische von der wäßrigen Phase trennt und
   (iv) optional den Wassergehalt der organischen Phase verringert,
dadurch gekennzeichnet, dass man die Extraktion in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

Die ethoxylierten Alkanole erfüllen die Formel

R¹-O-[-CH₂-CH₂-O-]ₘ-H

worin
R¹ geradkettiges oder verzweigtes bevorzugt geradkettiges C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
m eine rationale Zahl von 1 bis 3
   ist.

Beispiele für die zugrundeliegenden Alkanole R¹OH sind n-Octanol (Octylalkohol, Caprylalkohol), 2-Ethylhexanol, Nonylalkohol (Pelargonylalkohol), iso-Nonanol, n-Decanol, 2-Propylheptanol, Decylalkohol (Caprinalkohol), Undecylalkohol, Dodecylalkohol (Laurylalkohol), Tridecylalkohol und Tetradecylalkohol (Myristylalkohol).

In einer bevorzugten Ausführungsform handelt es sich um Reinsubstanzen wie beispielsweise 2-Ethylhexanol oder 2-Propylheptanol.

In einer weiteren Ausführungsform kann es sich bei dem zugrundeliegenden Alkanol R¹OH um ein Gemisch verschiedener Alkanole handeln, die im arithmetischen Mittel 8 bis 14, bevorzugt 9 bis 11 Kohlenstoffatome aufweisen. Da es sich um Gemische handelt, kann die Kohlenstoffzahl auch nicht-ganzzahlige Werte annehmen.

Beispiele für derartige Gemische sind Gemische von Fettalkoholen, z.B. solche, die aus Kokosöl erhalten worden sind. Solche Gemische sind überwiegend aus C₈- bis C₁₆-Alkanolen mit geradzahligen Kohlenstoffanzahlen zusammengesetzt, typischerweise 4,6-10,0 Gew% C₈-Alkanol, 5,0-8,0 Gew% C₁₀-Alkanol, 45,1-53,2 Gew% C₁₂-Alkanol, 16,8-21,0 Gew% C₁₄-Alkanol und 7,5-10,2 Gew% C₁₆-Alkanol.

In einer weiteren Ausführungsform handelt es sich bei dem Alkohol R¹-OH um ein Gemisch aus etwa 13 Kohlenstoffatome aufweisenden Alkoholen, besonders bevorzugt um ein solches, das durch Hydroformylierung aus einem C₁₂-Olefingemisch erhältlich ist, das seinerseits durch Oligomerisierung eines Olefingemisches erhältlich ist, das überwiegend vier Kohlenstoffatome aufweisende Kohlenwasserstoffe enthält.

Im statistischen Mittel weist dieses Olefingemisch 11 bis 16 Kohlenstoffatome auf, bevorzugt 11,1 bis 12,9, besonders bevorzugt 11,2 bis 12,8, ganz besonders bevorzugt 11,5 bis 12,5 und insbesondere 11,8 bis 12,2. Die daraus erhaltenen Alkohole weisen dementsprechend ein Kohlenstoffatom mehr auf.

In einer ganz besonders bevorzugten Ausführungsform weist dieser Alkohol R¹-OH einen mittleren Verzweigungsgrad, gemessen als ISO-Index, von 1,8 bis 2,7 auf.

Derartige Gemische sind als Tridecanole oder iso-Tridecanole kommerziell verfügbar.

Besonders bevorzugt handelt es sich um Gemische von linearen Alkoholen, die 9, 10 und 11 Kohlenstoffatome aufweisen. In einer ganz besonders bevorzugten Ausführungsform handelt es sich bei dem Alkanol R¹OH um ein Gemisch primärer Alkohole mit der Zusammensetzung 15-20 Gew% C₉-, 40-45 Gew% C₁₀- und 35 bis 40 Gew% C₁₁-Alkoholen, wobei der Anteil an Alkoholen mit 8 oder weniger bzw. mit 12 oder mehr Kohlenstoffatomen jeweils nicht mehr als 1 Gew% beträgt. Besonders bevorzugt handelt es sich dabei um hochlineare Alkanole, die einen Verzweigungsgrad, gemessen als ISO-Index, von nicht mehr als 0,5, bevorzugt nicht mehr als 0,3, besonders bevorzugt nicht mehr als 0,2 und ganz besonders bevorzugt nicht mehr als 0,1 aufweisen. Das durchschnittliche Molekulargewicht eines solchen Alkoholgemisches beträgt von 158 bis 164 g/mol. Die OH-Zahl beträgt von 342 bis 355 mg KOH/g.

Der Ethoxylierungsgrad m der ethoxylierten Alkanole ist eine rationale Zahl von 1 bis 3, bevorzugt 1,5 bis 3, besonders bevorzugt 1,75 bis 3, ganz besonders bevorzugt 1,75 bis 2,75 und insbesondere 2 bis 2,75.

Bei dem Ethoxylierungsgrad m handelt es sich um ein arithmetisches Mittel, daher kann m auch nicht-ganzzahlige Werte annehmen.

Die Oligo- und Polyethylenglykolen der Formel

HO-[-CH₂-CH₂-O]ₙ-H

werden zumeist bei der Ethoxylierung der Alkanole als Nebenprodukt gebildet.

Bei dem Polymerisationsgrad n handelt es sich ebenfalls eine rationale Zahl von mindestens 2, bevorzugt von 2 bis 30 und besonders bevorzugt von 2 bis 20. Auch hier handelt es sich um ein arithmetisches Mittel, daher kann m auch nicht-ganzzahlige Werte annehmen.

Der Anteil der Oligo- und Polyethylenglykole in den ethoxylierten Alkanolen variiert und ist zumeist abhängig von dem Wassergehalt in der Ethoxylierung und den Reaktionsbedingungen. Durch das geringe Molekulargewicht von Wasser reichen bereits geringe Mengen Wasser aus, um vergleichsweise hohe Gehalte an Oligo- und Polyethylenglykolen zu bewirken.

Quellen für Wasser sind besonders das eingesetzte Alkanol sowie die als Katalysator eingesetzte Base. Die Base wird in der Regel als wässrige Lösung eingesetzt, wobei eine anschliessende Entfernung nicht vollständig gelingt bzw. unökonomisch ist. Denkbar sind aber auch Luftfeuchtigkeit oder Feuchtigkeitsspuren in einem eventuell verwendeten Schutzgas, Kontaminierung in der eingesetzten Apparatur sowie ein geringer Wassergehalt in dem eingesetzten Ethylenoxid.

In der Regel kann der Gehalt an Oligo- und Polyethylenglykolen in den ethoxylierten Alkanolen bis zu 5 Gew% betragen, bevorzugt bis zu 3, besonders bevorzugt bis zu 2,5, ganz besonders bevorzugt bis zu 2, insbesondere bis zu 1,5 und speziell bis zu 1 Gew%.

Für unterschiedliche Anwendungen werden an den angestrebten Zielwert für den Gehalt an Oligo- und Polyethylenglykolen unterschiedliche Anforderungen gestellt, siehe unten.

Die Reaktion der Alkanole mit Ethylenoxid erfolgt zumeist unter Katalyse mit Basen, meist basischen (Erd)Alkalimetallsalzen.

Bei dem (Erd)Alkalimetall handelt es sich meist um Natrium, Kalium, Magnesium oder Calcium, bevorzugt Natrium oder Kalium, besonders bevorzugt Kalium.

Das Anion dieser basischen Salze ist ausgewählt aus der Gruppe bestehend aus Hydroxid, Oxid, Carbonat, Hydrogencarbonat, Phosphat, Hydrogenphosphat, Dihydrogenphosphat, C₁-C₁₀-Alkoholat und C₁-C₁₀-Carboxylat, bevorzugt Hydroxid, Oxid, Carbonat oder Hydrogencarbonat, besonders bevorzugt handelt es sich um Hydroxide.

Besonders bevorzugte basische (Erd)Alkalimetallsalze sind Natriumhydroxid, Natriumcarbonat, Natriumphosphat, Natriumacetat, Kaliumhydroxid, Kaliumcarbonat, Kaliumphosphat und Kaliumacetat, besonders bevorzugt sind Natriumhydroxid und Kaliumhydroxid.

Weiterhin bevorzugt sind die C₁-C₁₀-Alkoholate, bevorzugt C₁-C₄-Alkoholate, besonders bevorzugt Methanolate oder Ethanolate der (Erd)Alkalimetalle, besonders des Natrium oder Kalium.

In einer bevorzugten Ausführungsform wird das zugrundeliegende Alkanol R¹OH vor der Ethoxylierung in das korrespondierende (Erd)Alkalimetallalkanolat überführt, beispielsweise durch vorherige Umsetzung mit einem - methanolat oder -ethanolat oder Umsetzung mit dem betreffenden Metall, besonders Natrium oder Kalium.

Die Ethoxylierung erfolgt in der Regel, indem das Alkanol R¹OH und Ethylenoxid in einem Molverhältnis von 1 zu mindestens m umgesetzt werden. Besonders vorteilhaft ist ein Molverhältnis von 1 zu m bis 1 zu (1,5 × m), ganz besonders von 1 zu m bis 1 zu (1,25 × m), insbesondere von 1 zu m bis 1 zu (1,1 × m).

Die Umsetzung mit dem Ethylenoxid kann bei 50 bis 200 °C durchgeführt werden. Bevorzugt ist ein Temperaturbereich von 100 bis 180 °C, insbesondere von 120 bis 160 °C.

Die Umsetzung kann sowohl bei Atmosphärendruck, Unterdruck, als auch bei erhöhtem Druck, beispielsweise bei einem Druck von 0,8 bis 50 bar (abs), insbesondere bei einem Druck von 1 bis 10 bar (abs) durchgeführt werden. Speziell ist ein leichter Überdruck bis 2 bar (abs) vorteilhaft.

Das basische Salz wird in Mengen von 0,01 bis 5 Gew% bezogen auf das Alkanol R¹OH eingesetzt, bevorzugt 0,05 bis 2 und besonders bevorzugt 0,05 bis 0,5 Gew%.

Die Ethoxylierung wird bevorzugt unter einer Inertgasabdeckung durchgeführt, beispielsweise durch Stickstoff, Argon, Kohlenstoffdioxid oder Magerluft, also Sauerstoff-abgereicherter Luft, bevorzugt Stickstoff.

Im Inertgas können geringe, sicherheitstechnisch unbedenkliche Anteile molekularer Sauerstoff oder Stickstoffmonoxid anwesend sein, so dass die Explosionsgrenze des Ethylenoxids nicht überschritten wird, beispielsweise weniger als 5 Vol%, bevorzugt weniger als 2 Vol%, besonders bevorzugt weniger als 1 Vol% und ganz besonders bevorzugt weniger als 0,5 Vol%.

Es ist für das Verfahren nicht notwendig, Lösungsmittel für die Umsetzung zu verwenden. Es ist aber möglich, wenn auch weniger bevorzugt, das Verfahren in Gegenwart von organischen Lösungsmitteln wie beispielsweise aliphatischen, cycloaliphatischen oder aromatischen Kohlenwasserstoffen, Ethern, Acetalen, Ketonen, Estern oder cyclischen Carbonaten durchzuführen.

Wird das basische Salz als Feststoff eingesetzt, beispielsweise in Pulverform, so ist eine Reaktionsführung in Suspensionsfahrweise vorteilhaft, beispielsweise in einem oder mehreren Rührreaktoren.

Dabei kann das basische Salz entweder im Reaktor verbleiben, beispielsweise kann er durch eine Fritte, Filter oder ein Sieb zurückgehalten werden, oder kann mit dem Reaktoraustrag ausgetragen werden und anschließend vom Reaktionsgemisch abgetrennt werden, beispielsweise durch Sedimentieren, Filtrieren, Zentrifugieren oder Absorption, bevorzugt durch Filtration, was gegebenenfalls durch ein Filtrierhilfsmittel wie beispielsweise Celite, Aluminiumoxid, Silikate, Kieselgel oder Aktivkohle unterstützt werden kann.

Das basische Salz kann in Form einer wässrigen Lösung (in der Regel ca. 50%ig) zum Alkanol dosiert und anschließend das enthaltene Wasser im Vakuum bei erhöhter Temperatur entfernt werden, bis ein gewisser Wassergehalt (in der Regel 1000 ppm) unterschritten wird. Zu der so hergestellten Startermischung wird dann bei der jeweiligen Reaktionstemperatur Ethylenoxid dosiert.

Verfahren zur Ethoxylierung sind allgemein beschrieben in M. lonescu: Chemistry and Technology of Polyols for Polyurethanes, Rapra Technology Limited, 2005, ISBN:1-85957-491-2.

Die Umsetzung kann diskontinuierlich im Sinne eines Batch- oder Semi-Batch-Verfahrens oder kontinuierlich durchgeführt werden. Sie kann in einem Rührreaktor, Rohrreaktor, Schlaufenreaktor, in einem Festbettreaktor oder in einem Fließbettreaktor durchgeführt werden.

Es ist auch möglich, mehrere der genannten Reaktionsapparate hintereinander zu schalten. Dadurch kann das Verfahren in mehreren Verfahrensstufen betrieben werden. Es ist auch möglich, innerhalb einer Verfahrensstufe mehrere Reaktoren parallel zu betreiben.

Die Reaktionswärme kann hierbei beispielsweise über einen Reaktormantel, über aufgeschweißte Halbrohrschlangen oder Rohrschlangen, über Kühlrohre im Reaktor, nachfolgende oder vorgeschaltete Wärmetauscher, einen Totalkondensator bei Siedefahrweise, oder eine beliebige Kombination der genannten Varianten abgeführt werden.

Die Reaktionsmischung wird bei der kontinuierlichen Fahrweise im Kreislauf gefahren. Das erfolgt üblicherweise durch Umpumpen der Reaktionsmischung über einen äußeren Kreislauf. In diesen äußeren Kreislauf kann auch ein Wärmetauscher eingebaut sein.

Die Reaktionswärme kann hierbei beispielsweise über einen Reaktormantel, über aufgeschweißte Halbrohrschlangen oder Rohrschlangen, über Kühlrohre im Reaktor, nachfolgende oder vorgeschaltete Wärmetauscher, einen Totalkondensator bei Siedefahrweise, oder eine beliebige Kombination der genannten varianten abgeführt werden.

Das fertige ethoxylierte Alkanol wird durch Anlegen eines Vakuums sowie gegebenenfalls eines Strippgases, wie beispielsweise Stickstoff, Luft, Stickstoff-Luft-Gemische oder Wasserdampf von restlichem Ethylenoxid befreit. Anschließend wird das ethoxylierte Alkanol aufbereitet und von leicht flüchtigen Verunreinigungen befreit, vorzugsweise durch Strippen in einem Behälter oder einer Kolonne.

Ethoxylierte Alkanole, besonders die nach dem oben beschriebenen Verfahren erhältlichen ethoxylierten Alkanole, weisen je nach Wassergehalt der Einsatzstoffe einen Gehalt an Oligo- und Polyethylenglykolen auf, der bis zu 5 Gew% und mehr betragen kann.

Wenn die Herstellung in Gegenwart eines basischen Salzes eines (Erd)Alkalimetalls erfolgte, so kann das ethoxylierte Alkanol ferner auch Spuren dieses (Erd)Alkalimetalls enthalten, beispielsweise in Mengen bis zu 0,5 Gew%, bevorzugt bis zu 0,3. Besonders bevorzugt bis zu 0,2 und inbes bis zu 0,1 Gew%.

Je nach vorgesehener späterer Verwendung für das ethoxylierte Alkanol können sich der Gehalt an Oligo- und Polyethylenglykolen und/oder (Erd)Alkalimetall störend auswirken.

Dies ist bei den hydrophilen Oligo- und Polyethylenglykolen besonders dann der Fall, wenn es in der Anwendung der im Vergleich dazu relativ hydrophoben ethoxylierten Alkanole auf deren Hydrophobie ankommt, beispielsweise in der Anwendung in einem hydrophoben Medium, beispielsweise der Verteilung oder Dispergierung von Wasser in diesem hydrophoben Medium im Sinne einer w/o-Emulsion (Wasser in Öl).

Speziell Diethylenglykol als Oligo- und Polyethylenglykol ist Ausgangsprodukt für die Bildung von Dioxan, das aus toxikologischen Gründen aus dem Produkt abgetrennt werden sollte. Dioxan wird besonders unter sauren Bedingungen aus Diethylenglykol gebildet, beispielsweise bei der Herstellung von Polyethersulfaten aus ethoxylierten Alkanolen. Somit ist die erfindungsgemäße Abtrennung der Oligo- und Polyethylenglykole dann bevorzugt, wenn das ethoxylierte Alkanol in einer späteren Verwendung oder Derivatisierung sauren Bedingungen ausgesetzt ist, besonders bevorzugt wenn das ethoxylierte Alkanol später in das korrespondierende Polyethersulfat umgesetzt werden soll.

Es wurde beobachtet, dass bei Lagerung von ethoxylierten Alkanolen der Gehalt von Oligo- und Polyethylenglykol während der Lagerung steigt, besonders wenn die Lagerung unter einer Luftatmosphäre erfolgt. Dem kann entgegengewirkt werden, in dem die Lagerung unter einer sauerstoffabgereicherten Gasatmosphäre erfolgt und/oder man den Gehalt an Sauerstoff im ethoxylierten Alkanol vor Lagerung verringert.

Die Anwesenheit von (Erd)Alkalimetallen ist ebenfalls besonders bei Anwendungen in einem hydrophoben Medium möglichst gering zu halten, da diese Kationen dazu neigen, aus dem hydrophoben Medium auszufallen und so zu Ablagerungen führen können.

Dazu wird erfindungsgemäß das Gemisch aus Oligo- und Polyethylenglykolen und ethoxylierten Alkanolen einer Aufreinigung unterzogen, in der man
(i) das Gemisch mit einer bestimmten Menge Wasser bezogen auf die organische Phase vermischt,
(ii) die organische und wäßrige Phase entmischen lässt,
(iii) die organische von der wäßrigen Phase trennt und
(iv) optional den Wassergehalt der organischen Phase verringert.

Erfindungsgemäß führt man die Extraktion in Gegenwart einer Gasatmosphäre durch, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

Bei der Gasatmosphäre kann es sich um Sauerstoff-abgereicherter Luft (Magerluft) handeln oder ein unter den Extraktionsbedingungen inertes Gas, bevorzugt Stickstoff, Argon oder Kohlenstoffdioxid, besonders bevorzugt Stickstoff handeln.

Dazu wird mindestens einer der Schritte (i), (ii) oder (iii) in Gegenwart einer sauerstoffarmen Gasatmosphäre, bevorzugt mindestens einer der Schritte (ii) oder (iii), besonders bevorzugt mindestens der Schritt (ii), ganz besonders bevorzugt mindestens die Schritte (ii) und (iii) und insbesondere alle drei Schritte (i), (ii) und (iii).

In einer bevorzugten Ausführungsform werden solche Schritte in Gegenwart einer sauerstoffarmen Gasatmosphäre durchgeführt, in denen die Temperatur des ethoxylierten Alkanols mindestens 50 °C erreicht oder überschreitet.

Eine bevorzugte Ausführungsform der vorliegenden Erfindung besteht darin, den Gehalt an gelöstem Sauerstoff im ethoxylierten Alkanol und/oder im Wasser vor Einsatz in die Extraktion zu verringern, bevorzugt zumindest im ethoxylierten Alkanol, besonders bevorzugt sowohl im ethoxylierten Alkanol als auch im Wasser.

In einer besonders bevorzugten Ausführungsform erfolgt diese Verringerung des Gehalts an gelöstem Sauerstoff vor dem Erwärmen auf die Temperatur, bei der die Extraktion durchgeführt wird.

Die Verringerung des Gehalts an gelöstem Sauerstoff kann beispielsweise erfolgen durch Anlegen eines Vakuums und/oder Durchleiten eines Strippgases, wie bevorzugt das sauerstoffarme Gas oder Wasserdampf, bevorzugt durch Magerluft, Stickstoff, Argon oder Wasserdampf, besonders bevorzugt durch, Stickstoff oder Wasserdampf. Dabei wird bevorzugt ein Strippgas durch das jeweilige Medium geleitet, optional unterstützt durch Anlegen eines Vakuums.

Im Fall von Wasser kann der Verringerung des Gehalts an gelöstem Sauerstoff auch erreicht werden durch Erwärmen auf eine Temperatur von mindestens 50°C, bevorzugt mindestens 60°C, besonders bevorzugt mindestens 70°C und ganz besonders bevorzugt mindestens 80 °C. Ganz besonders bevorzugt wird das Wasser vor Kontakt mit dem ethoxylierten Alkanol auf die Temperatur erwärmt, bei der der Schritt (i) durchgeführt wird.

In einer bevorzugten Ausführungsform wird der Gehalt an gelöstem Sauerstoff in dem Gemisch aus ethoxyliertem Alkanol und Oligo- und Polyethylenglykolen vor Einsatz in die Extraktion um mindestens 20% gegenüber dem gesättigten Sauerstoffgehalt des Gemischs bei 20 °C und Normaldruck verringert, bevorzugt um mindestens 25%, besonders bevorzugt um mindestens 30, ganz besonders bevorzugt um mindestens 40, insbesondere um mindestens 50 und speziell um mindestens 60%. Es kann von Vorteil sein, den Sauerstoffgehalt sogar um mindestens 70, um mindestens 80 oder sogar um mindestens 90% zu verringern.

Da die Löslichkeit von Sauerstoff in dem Gemisch aus ethoxyliertem Alkanol und Oligo- und Polyethylenglykolen abhängig ist von der Lipophile bzw. Hydrophilie des Gemischs, also besonders von der Anzahl der Kohlenstoffatome im Alkanol, vom Ethoxylierungsgrad und vom Gehalt an Oligo- und Polyethylenglykolen, ist ein absoluter Gehalt an gelöstem Sauerstoff nur schwer anzugeben.

Es hat sich als vorteilhaft erwiesen, dass der Gehalt an gelöstem Sauerstoff in dem Gemisch aus ethoxyliertem Alkanol und Oligo- und Polyethylenglykolen vor Einsatz in die Extraktion nicht mehr als 100 Gew.ppm beträgt, bevorzugt nicht mehr als 90, besonders bevorzugt nicht mehr als 80, ganz besonders bevorzugt nicht mehr als 70, insbesondere nicht mehr als 60 und speziell nicht mehr als 50 Gew.ppm.

In Schritt (i) wird das Gemisch aus Oligo- und Polyethylenglykolen und ethoxylierten Alkanolen bei einer Temperatur von Umgebungstemperatur bis 90 °C mit dem 0,05 bis 2,0-fachen Volumen (v/v) Wasser je 1 Volumen an organischer Phase vermischt.

Die Temperatur bei der Vermischung reicht dabei von Umgebungstemperatur bis 90 °C, bevorzugt von 20 bis 85 °C, besonders bevorzugt von 35 bis 80 °C, ganz besonders bevorzugt von 40 bis 80 und insbesondere von 45 bis 75 °C.

Man kann auch bei höheren Temperaturen, beispielsweise bei 95 °C oder bei Anlegen von Überdruck sogar bei Temperatur von mehr als 100 °C arbeiten, dies ist jedoch weniger bevorzugt, da dies einerseits apparativ aufwendiger ist und andererseits die Stabilität des Produktes durch diese hohen Temperaturen beeinträchtigt werden kann.

Die Temperatur kann während der Vermischung gleich bleiben oder ansteigen.

Die Dauer der Vermischung ist weniger relevant, sie kann von 1 Minute bis 8 Stunden betragen, bevorzugt von 5 Minuten bis 4 Stunden, besonders bevorzugt von 10 Minuten bis 2 Stunden und ganz besonders bevorzugt von 15 bis 90 Minuten.

Erfindungsgemäß wesentlich im Vermischungsschritt (i) ist die Menge an Wasser, die vom 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase beträgt.

Geringere Volumina an Wasser vermögen die hydrophilen Bestandteile in einer wirtschaftlich vertretbaren Anzahl von Waschschritten nicht effektiv zu entfernen, größere Mengen an Wasser entfernen zu viel Wertprodukt aus dem Gemisch.

Das gemäß der vorliegenden Erfindung verwendete Wasser sollte bevorzugt ionenfrei sein, d.h. Wasser mit einem neutralen pH-Wert, das im Wesentlichen keine weiteren Ionen als die Hydroxid- und Hydroniumionen aus der Autoprotolyse von Wasser bei der jeweiligen Temperatur enthält.

Die elektrische Leitfähigkeit (bestimmt nach ASTM D 1125) bei 25 °C des verwendeten ionenfreien Wassers sollte vorzugsweise nicht mehr als 5 µS/cm, noch bevorzugter nicht mehr als 3, noch bevorzugter nicht mehr als 2 und insbesondere nicht mehr als 1 µS/cm betragen.

Bei dem verwendeten ionenfreien Wasser kann es sich um reines destilliertes oder zweifach destilliertes Wasser handeln oder um Wasser, das z.B. durch Ionenaustausch entionisiert wurde, vorzugsweise durch Ionenaustausch zumindest der Kationen, besonders bevorzugt durch Ionenaustausch sowohl der Kationen als auch der Anionen.

Die Vermischung der organischen Phase mit dem Wasser erfolgt in der Regel durch Energieeintrag durch Scherenergie. Dies kann beispielsweise in dynamischen Mischapparaten, d.h. durch Vermischen mittels Rührer oder durch Umpumpen (Naturumlauf oder Zwangsumlauf) bzw. Umpumpen mit statischen Mischorganen wie statischen Mischern oder Düsen im Umpumpkreis, durch statische Mischapparate wie statische Mischer, Düsen, Blenden, Y- oder T-Stücke im Zulauf des Ansatzbehälters, oder durch dynamische Mischapparate wie Mischpumpen oder Rührkessel erfolgen.

In Schritt (ii) werden die organische und die wässrige Phase bei erhöhter Temperatur entmischt, wobei ausgenutzt wird, dass die erfindungsgemäßen Systeme aus Oligo- und Polyethylenglykolen und ethoxylierten Alkanolen eine Obere Entmischungstemperatur (OET) aufweisen oder eine Emulsion bilden, die sich bei Erhöhung der Temperatur entmischt. In letzterem Fall ist es möglich, dass die Emulsionen durch die Anwesenheit ethoxylierter Alkanole mit einem Wert für m > 3 gebildet werden, da derartige höher ethoxylierte Alkanole, die sich produktionsbedingt zu einem geringen Anteil in den ethoxylierten Alkanolen befinden, als Emulgatoren wirken können.

Die Temperatur in Schritt (ii) wird in der Regel von 30 bis 90 °C gewählt, bevorzugt von 35 bis 85, besonders bevorzugt von 40 bis 85 und ganz besonders bevorzugt von 45 bis 80 °C.

Die Dauer der Entmischung kann von 10 Minuten bis 8 Stunden betragen, bevorzugt von 15 Minuten bis 4 Stunden, besonders bevorzugt von 30 Minuten bis 3 Stunden und ganz besonders bevorzugt von 45 Minuten bis 4 Stunden.

Bei einem hohen Gehalt an Oligo- und Polyethylenglykolen im System können diese als Löslichkeitsvermittler zwischen organischer und wässriger Phase wirken, was durch die Ausbildung von Mischphasen die Entmischung erschwert und verzögert. In diesem Fall kann die Beimischung eines organischen Lösungsmittels die Entmischung beschleunigen, das je nach geplanter Verwendung später im ethoxylierten Alkanol verbleiben oder aus diesem abgetrennt werden kann.

Denkbar, wenn auch weniger bevorzugt, ist die Verwendung von Emulsionsbrechern oder Phasentrennungshilfsmitteln zur Verbesserung oder Beschleunigung der Entmischung oder zur Stabilisierung der Phasengrenze, da diese zumeist im Produkt verbleiben. Bevorzugt werden derartige Hilfsmittel im erfindungsgemäßen Verfahren nicht eingesetzt.

Im Schritt (iii) trennt man die organische von der wäßrigen Phase.

Diese Trennung erfolgt in der Regel bei der gleichen Temperatur wie in Schritt (ii), sie kann jedoch auch ausnahmsweise bis zu 20, bevorzugt bis zu 15 und besonders bevorzugt bis zu 10 °C niedriger liegen.

Die Schritte (i) bis (iii) können beispielsweise in einem Rührbehälter oder in anderen herkömmlichen Apparaturen, z.B. in einer Kolonne oder Mixer-Settler-Apparatur, durchgeführt werden.

Verfahrenstechnisch können für diese Schritte im beschriebenen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Vorzugsweise werden Siebboden- oder gepackte beziehungsweise Füllkörperkolonnen, Rührbehälter oder Mixer-Settler-Apparate, sowie gepulste Kolonnen oder solche mit rotierenden Einbauten eingesetzt, besonders bevorzugt sind Rührbehälter und Mixer-Settler-Apparate.

Die Schritte (i) bis (iii) im erfindungsgemäßen Verfahren können ein- oder mehrmals durchgeführt werden, bevorzugt ein- bis zehnmal, besonders bevorzugt ein- bis achtmal und ganz besonders bevorzugt ein- bis sechsmal.

Eine häufigere Durchführung der Schritte (i) bis (iii) ist selbstverständlich möglich, ist jedoch zumeist unwirtschaftlich und führt zu erhöhten Verlusten an ethoxylierten Alkanolen als Wertprodukt.

Im optionalen Schritt (iv) kann der Wassergehalt der organischen Phase verringert werden.

Dies ist besonders dann bevorzugt, wenn das Produkt später zur Verteilung von Wasser in Kraftstoffen eingesetzt werden sollen, siehe unten.

Dazu kann das Produkt beispielsweise zur Wasserentfernung mit wasserbindenden Verbindungen, beispielsweise Zeolithe oder Molsieben behandelt werden oder einem Membranfiltrationsverfahren, insbesondere Ultrafiltration, Nanofiltration und Umkehrosmoseverfahren ausgesetzt werden. Die verwendeten Membranen haben die Eigenschaft, bestimmte Stoffe (wie organische Verbindungen) zurückzuhalten und andere (wie anorganische Salze oder Wasser) durchzulassen.

Bevorzugt wird in Schritt (iv) der Wassergehalt der organischen Phase jedoch per Destillation verringert, was optional durch Durchleiten eines Strippgases, wie bevorzugt das sauerstoffarme Gas, bevorzugt durch Magerluft, Stickstoff oder Argon, besonders bevorzugt durch Stickstoff unterstützt werden kann.

Bevorzugt wird auch Schritt (iv) in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

Bevorzugt ist es dabei, die thermische Belastung des Produktes während der Destillation von Wasser möglichst gering zu halten, indem man die Destillation über nicht mehr als 4 Stunden bei einer Temperatur von nicht mehr als 100 °C bei vermindertem Druck durchführt.

Bevorzugt wird die Destillation in weniger als 4 Stunden durchgeführt, besonders bevorzugt in nicht mehr als 3h45min, ganz besonders bevorzugt in nicht mehr als 3h30min.

Die Temperatur während der Destillation soll 100 °C nicht überschreiten, bevorzugt nicht mehr als 98 °C und besonders bevorzugt nicht mehr als 95 °C.

Die Destillation wird bei vermindertem Druck, also unter Umgebungsdruck durchgeführt, bevorzugt bei nicht mehr als 750 mbar, besonders bevorzugt bei nicht mehr als 500, ganz besonders bevorzugt nicht mehr als 250, insbesondere nicht mehr als 200 und speziell nicht mehr als 150 mbar.

Die Kombination aus Dauer, Temperatur und Druck werden so gewählt, dass der Wassergehalt in der organischen Phase auf nicht mehr als 5 Gew% verringert wird, bevorzugt nicht mehr als 4, besonders bevorzugt nicht mehr als 3, ganz besonders bevorzugt nicht mehr als 2 und insbesondere nicht mehr als 1 Gew%. Speziell können nicht mehr als 0,75 Gew% und sogar nicht mehr als 0,5, 0,25 oder 0,1 Gew% angestrebt werden.

Die Destillation kann in beliebiger Weise kontinuierlich oder diskontinuierlich durchgeführt werden. Bevorzugt erfolgt die destillative Abtrennung von Wasser in einem Rührkessel mit Doppelwandheizung und/oder innenliegenden Heizschlangen unter vermindertem Druck.

Selbstverständlich kann die Destillation auch durch ein oder mehrfaches Durchleiten durch einen Fallfilm-, Dünnschicht- oder Wischblattverdampfer erfolgen. Dazu wird das wässrige Gemisch, kontinuierlich oder diskontinuierlich, unter vermindertem Druck durch den Apparat geführt. Um die thermische Belastung des Destillationsrückstandes möglichst gering zu halten, wird der Ablauf nach Durchlaufen des Verdampfers bevorzugt abgekühlt.

Vorteilhaft kann ein inertes Gas, bevorzugt Argon oder ein stickstoffhaltiges Gas, besonders bevorzugt Argon, Stickstoff oder ein Gemisch aus Luft und Stickstoff (Magerluft), ganz besonders bevorzugt Stickstoff durch den Destillationsapparat ein- oder übergeleitet werden, beispielsweise 0,1 - 1, bevorzugt 0,2 - 0,8 und besonders bevorzugt 0,3 - 0,7 m³/m³h, bezogen auf das Volumen des flüssigen Gemisches.

Den Destillationsapparaten kann optional auch eine Rektifikationskolonne mit bis zu 10 theoretischen Böden aufgesetzt werden, dies ist jedoch bei der einfachen Abtrennung von Wasser in der Regel nicht erforderlich und daher weniger bevorzugt.

Durch die Durchführung des erfindungsgemäßen Verfahrens ist es möglich, den Anfangsgehalt an Oligo- und Polyethylenglykolen in den ethoxylierten Alkanolen in der Regel um mindestens 10%, bevorzugt mindestens um 15, besonders bevorzugt um mindestens 20 und ganz besonders bevorzugt um mindestens 25% zu verringern. Durch das erfindungsgemäße Verfahren ist es möglich, die Oligo- und Polyethylenglykole nahezu vollständig zu entfernen und deren Bildung während des Verfahrens zu verringern.

Der Anfangsgehalt an (Erd)Alkalimetallionen in den ethoxylierten Alkanolen kann in der Regel um mindestens 20%, bevorzugt mindestens um 30, besonders bevorzugt um mindestens 40 und ganz besonders bevorzugt um mindestens 50% und bis zu 95% oder mehr verringert werden.

Die nach dem erfindungsgemäßen Verfahren erhältlichen, bevorzugt erhaltenen, an Oligo- und Polyethylenglykolen und/oder (Erd)alkalimetallionen abgereicherten ethoxylierten Alkanole können allgemein in allen Anwendungen eingesetzt werden, die typischerweise für derartige ethoxylierte Alkanole bekannt sind, die nicht abgereichert sind.

Diese können beispielsweise sein der Einsatz als
- Netzmittel
- Detergenz
- Dispergiermittel
- Lösungsvermittler
- zur Textilreinigung und Textilfärbung
- Intermediat zur Synthese anionischer Phosphate, Sulfate oder Ethercarboxylaten
- als Adjuvans in Agroformulierungen: Hilfsmittel zur Verbesserung der

- - Haftung
- - Retention (engl. spray retention aid)
- - Verteilung
- - Penetration
- - Benetzungshilfsmittel (Spreizmittel, engl. spreading aid) von Spritzbrühen.

Mit Vorzug werden die ethoxylierten Alkanole jedoch in solchen Anwendungen eingesetzt, in denen ein verringerter Gehalt an Oligo- und Polyethylenglykolen und/oder (Erd)alkalimetallionen gefordert wird.

Dies können beispielsweise Emulgatoren in w/o-Emulsionen sein.

Mit besonderem Vorzug werden diese ethoxylierten Alkanole mit einem verringerten Gehalt an Oligo- und Polyethylenglykolen und/oder (Erd)alkalimetallionen eingesetzt als oberflächenaktives Hilfsmittel zur Verteilung, besonders Dispergierung von Wasser in Kraftstoffen, ausgewählt aus der Gruppe bestehend aus Benzin, Diesel, marinen Kraftstoffen und Luftfahrtkraftstoffen, bevorzugt Diesel- oder Luftfahrtkraftstoffe, besonders bevorzugt Luftfahrtkraftstoffe, besonders Turbinenkraftstoffe.

Mit diesen ethoxylierten Alkanolen ist es möglich, einen Gehalt an Wasser von mindestens 50 ppm in flüssigen Kohlenwasserstoffkraftstoffen in stabilen Mikroemulsionen mit einer Tröpfchengröße von nicht mehr als 0,25 µm zu verteilen. Dies hat besonders den Zweck, bei Abkühlung bis zu minus 50°C die Bildung von Eispartikeln in dem Kraftstoff zu verringern oder zu unterdrücken. Zudem wird die Bildung einer separierten Wasserphase am Tankboden von Flugzeugen verhindert, die u.a. zu unerwünschter Korrosion infolge von Biofilmbildung führt.

Dazu können die ethoxylierten Alkanole allein oder bevorzugt in Kombination mit einem (C₈-C₂₄)alkylamido (C₁-C₆)alkyl betain eingesetzt werden.

Eine derartige Anwendung und entsprechende Gemische sind beschrieben in WO 2011/045334 A1.

Zur Verringerung der Eiskristallbildung werden dem Kraftstoff bevorzugt folgende Mengen zugesetzt:
- von 45 bis 4575 ppm, bevorzugt 45 bis 500 ppm mindestens eines ethoxylierten Alkanols und/oder
- von 0 bis 425 ppm, z.B. 1 bis 425 ppm, bevorzugt 1 bis 5 ppm mindestens eines (C₈-C₂₄)alkylamido (C₁-C₆)alkyl betains.

Bei dem mindestens einen (C₈-C₂₄)alkylamido (C₁-C₆)alkyl betain kann es sich bevorzugt um Cocoamidopropyl betain handeln.

Zusätzlich zu diesem oberfächenaktiven Mittel oder Emulgator kann der Kraftstoff noch eines oder mehrere der folgenden zusätzlichen Komponenten enthalten: Statikdissipatoren, Antioxidantien, Metalldeaktivatoren, Lecksuchadditive, Korrosionsinhibitoren, Schmiermittel, Alkohole, Glykole und andere Standardprodukte, die dem Fachmann bekannt sind, sowie Verunreinigungen wie Fettsäuremethylester.

Die ethoxylierten Alkanole werden zumeist in Form von flüssigen Konzentraten eingesetzt, im wesentlichen enthaltend
0,1 bis 10 Gew% mindestens eines (C₈-C₂₄)alkylamido (C₁-C₆)alkyl betains, bevorzugt Cocoamidopropyl betain
30 bis 95 Gew% mindestens eines ethoxylierten Alkanol, mit einem verringerten Gehalt an Oligo- und Polyethylenglykolen und/oder (Erd)alkalimetallionen
0 bis 20 Gew% mindestens eines Glycol-basierten Löslichkeitsvermittlers, bevorzugt Ethylenglykol, und
0 bis 65 Gew% mindestens eines organischen Lösungsmittels, bevorzugt Ethanol.

Analog WO 2011/045334 A1 ist eine bevorzugte Zusammensetzung des flüssigen Konzentrats
2 Teile Cocoamidopropyl betain
60 Teile mindestens eines ethoxylierten Alkanol, mit einem verringerten Gehalt an Oligo- und Polyethylenglykolen und/oder (Erd)alkalimetallionen
4 Teile Ethylenglykol, und
34 Teile Ethanol.

Die Turbinenkraftstoffe enthalten eine Hauptmenge eines flüssigen Turbinenkraftstoffs, wobei es sich beispielsweise um einen in der zivilen oder militärischen Luftfahrt üblichen Turbinenkraftstoff handelt. Dazu zählen beispielsweise Kraftstoffe der Bezeichnung Jet Fuel A, Jet Fuel A-1, Jet Fuel B, Jet Fuel JP-4, JP-5, JP-7, JP-8 und JP-8+100. Jet A und Jet A-1 sind kommerziell erhältliche Turbinenkraftstoffspezifikationen auf Kerosinbasis. Die zugehörigen Normen sind ASTM D 1655 sowie DEF STAN 91-91. Jet B ist ein weiter geschnittener Kraftstoff auf Basis von Naphtha- und Kerosinfraktionen. JP-4 ist äquivalent zu Jet B. JP-5, JP-7, JP-8 und JP-8+100 sind militärische Turbinenkraftstoffe, wie sie beispielsweise von der Marine und Luftwaffe eingesetzt werden. Zum Teil bezeichnen diese Normen Formulierungen, die bereits weitere Additive, wie Korrosionsinhibitoren, Vereisungsinhibitoren, statische Dissipatoren, etc. enthalten.

Dem Turbinenkraftstoff können weitere an sich bekannten Zusatzstoffe zugegeben werden. Geeignete Zusatzstoffe, die in der Turbinenkraftstoffzusammensetzung enthalten sein können, umfassen üblicherweise Detergenzien, Korrosionsinhibitoren, schwefelfreie Antioxidantien wie sterisch gehinderte tert.-Butylphenole, N-Butylphenylendiamine oder N,N'-Diphenyl-amin und Derivate hiervon, Metalldesaktivatoren wie N,N'-Disalicyliden-1,2-diamino-propan, Lösungsvermittler, Antistatika wie Stadis 450, Biocide, Anti-Icing-Mittel wie Diethylen-glykolmethylether oder Triethylenglykolmethylether, sowie Mischungen der genannten Zusatzstoffe.

Spezifikationen für Turbinenkraftstoff sowie zugelassene Zusatzstoffe mit den jeweiligen Dosierungen sind aufgeführt in ASTM D1655-24.

Die Mikroemulsionen können hergestellt werden durch Mischen von
- 99,995 bis 99,999 Teile, z.B. 99,998 Teile, eines Kraftstoffs, z.B. einem Turbinenkraftstoff und
- etwa 0,0001 bis etwa 0,01 Teile, z.B. 0,025 Teile, Emulgatorzusammensetzung, wobei die Emulgatorzusammensetzung umfassen

- - mindestens eines (C₈-C₂₄)alkylamido (C₁-C₆)alkyl betains, bevorzugt Cocoamidopropyl betain und
- - mindestens eines ethoxylierten Alkanols, mit einem verringerten Gehalt an Oligo- und Polyethylenglykolen und/oder (Erd)alkalimetallionen,
wobei sich die Angaben der Teile stets auf das Volumen beziehen.

Demzufolge ist ein weiterer Gegenstand der vorliegenden Erfindung ein Verfahren zur Verteilung von Wasser in Kraftstoffen, mit Hilfe von ethoxylierten Alkanolen der Formel

R¹-O-[-CH₂-CH₂-O-]ₘ-H

worin
R¹ geradkettiges oder verzweigtes C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
m eine rationale Zahl von 1 bis 3
mit einem verringerten Gehalt an Oligo- und Polyethylenglykolen der Formel

   HO-[-CH₂-CH₂-O]ₙ-H

   worin n eine rationale Zahl von mindestens 2, bevorzugt von 2 bis 30 und besonders bevorzugt von 2 bis 20 ist,
   und einem gleichzeitig verringerten Gehalt an (Erd)Alkalimetallionen,
   worin man
      (I) ein Alkanol R¹-OH mit mindestens m Äquivalenten Ethylenoxid in Gegenwart mindestens eines basischen Salzes mindestens eines (Erd)Alkalimetalls sowie in Gegenwart von Wasser bei einer Temperatur von 20 bis 200 °C unter Erhalt eines Gemisches aus Oligo- und Polyethylenglykolen und ethoxylierten Alkanolen miteinander reagieren lässt und
      (II) das so erhaltene Gemisch einer Aufreinigung unterwirft, in der man
         (i) das Gemisch aus Oligo- und Polyethylenglykolen und ethoxylierten Alkanolen bei einer Temperatur von Umgebungstemperatur bis 90 °C mit dem 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase vermischt,
         (ii) die Phasen bei einer Temperatur von 30 bis 90 °C entmischen lässt,
         (iii) die organische von der wäßrigen Phase trennt und
         (iv) optional den Wassergehalt der organischen Phase verringert,
      (III) das so erhaltene Oligo- und Polyethylenglykol-abgereicherte, ethoxylierte Alkanol in einen Kraftstoff mischt, ausgewählt aus der Gruppe bestehend aus Benzin, Diesel, marine Kraftstoffe und Luftfahrtkraftstoffe.

### Beispiele

### Material und Methoden

Im Mittel 2,5-fach ethoxyliertes Gemisch von C₉-C₁₁-Alkanolen, beispielsweise Synperonic^{™} 91/2.5 von Croda, CAS 68439-46-3.

### Kaliumgehalte wurde mittels ICP-OES gemessen

PEG-Gehalte wurden mittels HPLC-MS gegen PEG-Standard gemessen. Es wurde PEG-Standard des jeweiligen Molmassenbereichs verwendet, der in der Probe mittels MS bestimmt wurde.

Wassergehalte wurden mittels volumetrischer Karl Fischer Titration gemessen.

Die Versuche wurden in Doppelmantelreaktoren mit mehrstufigem Schrägblattrührer, Thermostat und Bodenablauf durchgeführt. Die Phasentrennungen erfolgten durch Ablassen der wässrigen Phase durch das Bodenablassventil.

### Beispiel 1

Gemäß ASTM D 1094 - 07 wurden 80 ml eines Luftfahrtkraftstoffs der Spezifikation Jet A-1 bei Umgebungstemperatur mit 20 ml pH 7 Pufferlösung und der in der Tabelle angegebenen Menge bezogen auf den Kraftstoff an Additiv und Polyethylenglykol des mittleren Molgewichts 400 g/mol (PEG400) geschüttelt und die Phasen sowie deren Entmischung in einem 100 ml Standzylinder nach einem Zeitraum von 5, 30 und 60 Minuten bewertet.

Bei dem Additiv handelt es sich um die Zusammensetzung gemäß Beispiel 4 der WO 2011/045334 A1, wobei als ethoxylierter Alkanol ein im Mittel 2,5-fach ethoxyliertes Gemisch von C₉-C₁₁-Alkanolen eingesetzt wurde.

**Tabelle 1**

| | | | Abtrennung Wasser nach | | |
|---|---|---|---|---|---|
| Eintrag | Additiv [ppm] | PEG400 [ppm] | 5 min | 30 min | 60 min |
| 0 | 0 | 0 | 19 ml^{[1]} | 19 mL Wasser^{[1]} | 19mL Wasser^{[1]} |
| 1 | 1000 | 50 | 20 ml | 4ml Mischphase | 4ml Mischphase |
| 2 | 1000 | 0 | 20 ml | 3ml Mischphase | 3ml Mischphase |
| 3 | 1000 | 200 | 20 ml | 4ml Mischphase | 4ml Mischphase |
| 4 | 250 | 0 | 20 ml | 0ml Mischphase | 0ml Mischphase |
| 5 | 250 | 100 | 20 ml | 2ml Mischphase | 1,5ml Mischphase |

| | | | | | |
|---|---|---|---|---|---|
| [1] einzelne stabile Wasserstropfen in organischer Phase | | | | | |

Bei der Mischphase handelt es sich um eine trübe Phase zwischen der wässrigen und der organischen Phase.

Man sieht, dass das Additiv ohne Anwesenheit von Polyethylenglykol (Einträge 2 und 4) eine gute Phasentrennung ohne bzw. mit nur wenig Mischphase bewirkt.

Die Anwesenheit von Polyethylenglykol bewirkt eine Zunahme der Mischphase (Einträge 1 und 3 verglichen mit 2 und Eintrag 5 verglichen mit 4).

Die Anwesenheit von Polyethylenglykol hat also eine Verschlechterung der Phasentrennung zwischen Wasser und Luftfahrtkraftstoff zur Folge.

### Beispiel 2

500 g ethoxyliertes Alkanol (PEG-Gehalt: 0,92 Gew%, Kaliumgehalt: 0,1 Gew%, Wassergehalt: 0,3 Gew%) und 250 ml entsalztes Wasser wurden unter einer Stickstoffatmosphäre auf 80°C erhitzt und 30 min. bei dieser Temperatur gerührt.

Die wässrige Phase wurde bei 80°C abgetrennt. 250 ml Wasser wurden zugegeben und 30 min bei 80°C gerührt. Die wässrige Phase wurde abgetrennt. Das Ethoxylat wurde auf diese Weise weitere dreimal mit jeweils 250 ml entsalztem Wasser extrahiert (insgesamt wurde fünfmal mit je 250 ml entsalztem Wasser extrahiert).

Nach der letzten Phasentrennung enthielt die organische Phase (549 g) 11,8% Wasser. Das Wasser wurde mittels eines Rotationsverdampfers bei 100°C unter Vakuum entfernt. Das Endprodukt zeigte einen Wassergehalt von 0,2%, einen Kaliumgehalt von 4 ppm und einen PEG-Gehalt von <50 ppm.

### Beispiel 3

Dieser Versuch wurde unter Luftatmosphäre durchgeführt und das ethoxylierte Alkanol wurde nicht entgast:
1000 g eines 2,5-fach ethoxylierten Gemischs von C₉-C₁₁-Alkanolen (PEG-Gehalt 109 ppm) wurden auf 80°C erhitzt, mit 500 g entsalztem Wasser versetzt, und die Mischung wurde anschließend 1 h bei 80°C gerührt. Man ließ die Phasen über 17 h trennen und trennte die wässrige Phase ab (332,5 g, wässrige Phase 1). Man gab weitere 500 g entsalztes Wasser zu, rührte 1 h bei 80°C, ließ die Phasen für 1,5 h trennen und trennte die wässrige Phase ab (490,8 g, wässrige Phase 2). Aus der organischen Phase wurde das Wasser im Vakuum abdestilliert (80°C/100 mbar).

Man erhielt 980 g ethoxyliertes Alkanol mit einem Wassergehalt von 0,01%.

Die folgenden PEG-Gehalte wurden bestimmt:

| Phase | Masse [g] | PEG Gehalt [ppm] | Masse PEG [mg] |
|---|---|---|---|
| Ausgangsverbindung | 1000 | 109 | 109 |
| wässrige Phase 1 | 332,5 | 371 | 123 |
| wässrige Phase 2 | 490,8 | 125 | 61 |
| ethoxyliertes Alkanol | 980 | 120 | 118 |

Die Massenbilanz ergibt, dass das ethoxylierte Alkanol zu Beginn des Versuchs 109 mg PEG enthielt, und die 3 Phasen am Ende des Versuchs in Summe 302 mg PEG. Im Laufe des Versuchs wurden entsprechend 193 mg PEG neu gebildet (177% in Bezug auf die ursprüngliche Menge). Der Versuch zeigt weiterhin, dass bei Anwesenheit von Sauerstoff der PEG-Gehalt durch Extraktion nicht gesenkt werden kann.

### Beispiel 4:

Dieser Versuch wurde unter Stickstoffatmosphäre durchgeführt und das ethoxylierte Alkanol wurde entgast, indem ein Stickstoffstrom für 30 min. durchgeleitet wurde:
400 g eines 2,5-fach ethoxylierten Gemischs von C₉-C₁₁-Alkanolen (PEG-Gehalt 109 ppm) wurden auf 80°C erhitzt, mit 200 g entsalztem Wasser versetzt, und die Mischung wurde anschließend 30 min. bei 80°C gerührt. Man ließ die Phasen über 1,5 h trennen.

Eine Probe aus der organischen Phase zeigte einen Wassergehalt von 18,2% und einen PEG-Gehalt von 50 ppm. Berechnet auf das wasserfreie ethoxylierte Alkanol wurde der PEG-Gehalt entsprechend auf 61 ppm reduziert. Dieser Versuch zeigt im Vergleich zu Beispiel 3, dass Sauerstoffausschluss bei der Extraktion zu einer Verringerung des PEG-Gehalts des ethoxylierten Alkanols führen kann.

### Beispiel 5:

Dieser Versuch wurde unter Stickstoffatmosphäre durchgeführt und das ethoxylierte Alkanol sowie das entsalzte Wasser wurden entgast, indem ein Stickstoffstrom für 15 h durchgeleitet wurde:
600 g eines 2,5-fach ethoxylierten Gemischs von C₉-C₁₁-Alkanolen (PEG-Gehalt 109 ppm) wurden auf 80°C erhitzt, mit 300 g entsalztem Wasser versetzt, und die Mischung wurde anschließend 30 min. bei 80°C gerührt. Man ließ die Phasen über 2 h trennen und trennte die wässrige Phase ab (186 g), die 249 ppm PEG enthielt. Die organische Phase (714 g, 15,7% Wasser) enthielt 34 ppm PEG.

| Phase | Masse [g] | PEG Gehalt [ppm] | Masse PEG [mg] |
|---|---|---|---|
| Ausgangsverbindung | 600 | 109 | 65 |
| wässrige Phase | 186 | 249 | 47 |
| ethoxyliertes Alkanol | 714 | 34 | 24 |

Die Massenbilanz ergibt, dass die 2 Phasen am Ende des Versuchs in Summe 71 mg PEG enthielten, während das ethoxylierte Alkanol zu Beginn des Versuchs 65 ppm PEG enthielt. Im Laufe des Versuchs wurden entsprechend 6 mg PEG neu gebildet (9% in Bezug auf die ursprüngliche Menge).

## Patentansprüche

1. Verfahren zur Abtrennung von Oligo- und Polyethylenglykolen der Formel
HO-[-CH₂-CH₂-O]ₙ-H
worin n eine rationale Zahl von mindestens 2, bevorzugt von 2 bis 30 und besonders bevorzugt von 2 bis 20 ist
aus ethoxylierten Alkanolen der Formel
R¹-O-[-CH₂-CH₂-O-]ₘ-H
worin
R¹ geradkettiges oder verzweigtes, bevorzugt geradkettiges C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
m eine rationale Zahl von 1 bis 3
ist, wobei man
(i) das Gemisch aus Oligo- und Polyethylenglykolen und ethoxylierten Alkanolen bei einer Temperatur von Umgebungstemperatur bis 90 °C mit dem 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase vermischt,
(ii) die Phasen bei einer Temperatur von 30 bis 90 °C entmischen lässt,
(iii) die organische von der wäßrigen Phase trennt,
(iv) optional den Wassergehalt der organischen Phase verringert,
**dadurch gekennzeichnet, dass** man die Extraktion in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

2. Verfahren gemäß Anspruch 1 zur gleichzeitigen Abtrennung von Oligo- und Polyethylenglykolen und (Erd)alkalimetallionen aus ethoxylierten Alkanolen, die durch Umsetzung eines Alkanols R¹-OH mit mindestens m Äquivalenten Ethylenoxid in Gegenwart mindestens eines basischen Salzes mindestens eines (Erd)Alkalimetalls erhalten worden sind.

3. Verfahren zur Herstellung von ethoxylierten Alkanolen der Formel
R¹-O-[-CH₂-CH₂-O-]ₘ-H
worin
R¹ geradkettiges oder verzweigtes C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
m eine rationale Zahl von 1 bis 3
mit einem verringerten Gehalt an Oligo- und Polyethylenglykolen der Formel
HO-[-CH₂-CH₂-O]ₙ-H
worin n eine rationale Zahl von mindestens 2, bevorzugt von 2 bis 30 und besonders bevorzugt von 2 bis 20 ist,
und einem gleichzeitig verringerten Gehalt an (Erd)Alkalimetallionen,
wobei man
(I) ein Alkanol R¹-OH mit mindestens m Äquivalenten Ethylenoxid in Gegenwart mindestens eines basischen Salzes mindestens eines (Erd)Alkalimetalls sowie in Gegenwart von Wasser bei einer Temperatur von 20 bis 200 °C unter Erhalt eines Gemisches aus Oligo- und Polyethylenglykolen und ethoxylierten Alkanolen miteinander reagieren lässt und
(II) das so erhaltene Gemisch einer Aufreinigung unterwirft, in der man
(i) das Gemisch aus Oligo- und Polyethylenglykolen und ethoxylierten Alkanolen bei einer Temperatur von Umgebungstemperatur bis 90 °C mit dem 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase vermischt,
(ii) die Phasen bei einer Temperatur von 30 bis 90 °C entmischen lässt,
(iii) die organische von der wäßrigen Phase trennt,
(iv) optional den Wassergehalt der organischen Phase verringert,
**dadurch gekennzeichnet, dass** man die Extraktion in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

4. Verfahren gemäß Anspruch 1 oder 3, **dadurch gekennzeichnet, daß** man mindestens einen der Schritte (i), (ii) oder (iii) in Gegenwart einer sauerstoffarmen Gasatmosphäre durchführt, bevorzugt mindestens einen der Schritte (ii) oder (iii), besonders bevorzugt mindestens der Schritt (ii), ganz besonders bevorzugt mindestens die Schritte (ii) und (iii) und insbesondere alle drei Schritte (i), (ii) und (iii).

5. Verfahren gemäß einem der vorstehenden Ansprüche, **dadurch gekennzeichnet, dass** man solche Reaktionsschritte in Gegenwart einer sauerstoffarmen Gasatmosphäre durchführt, in denen die Temperatur des ethoxylierten Alkanols mindestens 50 °C erreicht oder überschreitet.

6. Verfahren gemäß einem der vorstehenden Ansprüche, **dadurch gekennzeichnet, daß** man die Schritte (i) bis (iii) ein- bis zehnmal durchläuft, bevorzugt ein- bis achtmal, besonders bevorzugt ein- bis sechsmal.

7. Verfahren gemäß einem der vorstehenden Ansprüche, **dadurch gekennzeichnet, daß** man den Gehalt an gelöstem Sauerstoff im ethoxylierten Alkanol und/oder im Wasser vor Einsatz in die Extraktion verringert, bevorzugt zumindest im ethoxylierten Alkanol, besonders bevorzugt sowohl im ethoxylierten Alkanol als auch im Wasser.

8. Verfahren gemäß Anspruch 7, **dadurch gekennzeichnet, dass** man den Gehalt an gelöstem Sauerstoff im ethoxylierten Alkanol die Extraktion durch Anlegen eines Vakuums und/oder Durchleiten eines Strippgases, wie bevorzugt das sauerstoffarme Gas oder Wasserdampf, verringert.

9. Verfahren gemäß Anspruch 7, **dadurch gekennzeichnet, dass** der Gehalt an gelöstem Sauerstoff im Gemisch aus ethoxyliertem Alkanol und Oligo- und Polyethylenglykolen vor Einsatz in die Extraktion um mindestens 20% gegenüber dem Sättigungssauerstoffgehalt des Gemischs bei 20 °C und Normaldruck verringert wird.

10. Verfahren gemäß Anspruch 7, **dadurch gekennzeichnet, dass** man den Gehalt an gelöstem Sauerstoff im Wasser durch Erwärmen auf eine Temperatur von mindestens 50°C verringert.

11. Verfahren gemäß einem der vorstehenden Ansprüche, **dadurch gekennzeichnet, daß** man zusätzlich Schritt (iv) in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt.

12. Verfahren gemäß einem der vorstehenden Ansprüche, **dadurch gekennzeichnet, daß** es sich bei dem Alkanol R¹-OH um ein Gemisch aus etwa 13 Kohlenstoffatome aufweisenden Alkoholen handelt, das durch Hydroformylierung aus einem C₁₂-Olefingemisch erhältlich ist und einen mittleren Verzweigungsgrad, gemessen als ISO-Index, von 1,8 bis 2,7 aufweist.

13. Verfahren gemäß einem der Ansprüche 1 bis 16, **dadurch gekennzeichnet, daß** es sich bei dem Alkanol R¹-OH um ein Gemisch primärer Alkohole handelt mit der Zusammensetzung 15-20 Gew% C₉-, 40-45 Gew% C₁₀- und 35 bis 40 Gew% C₁₁-Alkoholen, wobei der Anteil an Alkoholen mit 8 oder weniger bzw. mit 12 oder mehr Kohlenstoffatomen jeweils nicht mehr als 1 Gew% beträgt.

14. Verwendung von Oligo- und Polyethylenglykol- sowie optional (Erd)Alkalimetall-abgereicherten ethoxylierten Alkanolen erhalten nach einem der vorstehenden Verfahren zur Verteilung von Wasser in Kraftstoffen, besonders Benzin, Diesel, marine Kraftstoffe und Luftfahrtkraftstoffe.

15. Verwendung von Oligo- und Polyethylenglykol- sowie optional (Erd)Alkalimetall-abgereicherten ethoxylierten Alkanolen erhalten nach einem der Verfahren gemäß einem der Ansprüche 1 bis 11 als
- Netzmittel
- Detergenz
- Dispergiermittel
- Lösungsvermittler
- zur Textilreinigung und Textilfärbung
- Intermediat zur Synthese anionischer Phosphate, Sulfate oder Ethercarboxylaten
- als Adjuvans in Agroformulierungen: Hilfsmittel zur Verbesserung der
- - Haftung
- - Retention (engl. spray retention aid)
- - Verteilung
- - Penetration
- - Benetzungshilfsmittel (Spreizmittel, engl. spreading aid) von Spritzbrühen.

16. Verfahren zur Verteilung von Wasser in Kraftstoffen, mit Hilfe von ethoxylierten Alkanolen der Formel
R¹-O-[-CH₂-CH₂-O-]ₘ-H
worin
R¹ geradkettiges oder verzweigtes C₈-C₁₄-Alkyl, bevorzugt C₈-C₁₃-Alkyl, besonders bevorzugt C₉-C₁₃-Alkyl, und ganz besonders bevorzugt C₉-C₁₁-Alkyl und
m eine rationale Zahl von 1 bis 3
mit einem verringerten Gehalt an Oligo- und Polyethylenglykolen der Formel
HO-[-CH₂-CH₂-O]ₙ-H
worin n eine rationale Zahl von mindestens 2, bevorzugt von 2 bis 30 und besonders bevorzugt von 2 bis 20 ist,
und einem gleichzeitig verringerten Gehalt an (Erd)Alkalimetallionen,
worin man
(I) ein Alkanol R¹-OH mit mindestens m Äquivalenten Ethylenoxid in Gegenwart mindestens eines basischen Salzes mindestens eines (Erd)Alkalimetalls sowie in Gegenwart von Wasser bei einer Temperatur von 20 bis 200 °C unter Erhalt eines Gemisches aus Oligo- und Polyethylenglykolen und ethoxylierten Alkanolen miteinander reagieren lässt und
(II) das so erhaltene Gemisch einer Aufreinigung unterwirft, in der man
(i) das Gemisch aus Oligo- und Polyethylenglykolen und ethoxylierten Alkanolen bei einer Temperatur von Umgebungstemperatur bis 90 °C mit dem 0,05 bis 2,0-fachen, bevorzugt 0,1 bis 1,5-fachen, besonders bevorzugt 0,15 bis 1,0-fachen, ganz besonders bevorzugt 0,25 bis 0,75-fachen Volumen (v/v) Wasser je 1 Volumen an organischer Phase vermischt,
(ii) die Phasen bei einer Temperatur von 30 bis 90 °C entmischen lässt,
(iii) die organische von der wäßrigen Phase trennt und
(iv) optional den Wassergehalt der organischen Phase verringert,
wobei man die Extraktion in Gegenwart einer Gasatmosphäre durchführt, in der der Anteil an Sauerstoff nicht mehr als 10 Vol%, bevorzugt nicht mehr als 8, besonders bevorzugt nicht mehr als 6, ganz besonders bevorzugt nicht mehr als 5, insbesondere nicht mehr als 2 und speziell nicht mehr als 1 Vol% beträgt,
(III) das so erhaltene Oligo- und Polyethylenglykol-abgereicherte, ethoxylierte Alkanol in einen Kraftstoff mischt, ausgewählt aus der Gruppe bestehend aus Benzin, Diesel, marine Kraftstoffe und Luftfahrtkraftstoffe.

17. Kraftstoff, enthaltend ein Oligo- und Polyethylenglykol-abgereichertes, ethoxyliertes Alkanol erhalten gemäß einem Verfahren gemäß einem der Ansprüche 1 bis 11.
